(19) Europäisches Patentamt
European Patent Office
Office européen des brevets

(11) EP 0 515 420 B1

(12) # EUROPÄISCHE PATENTSCHRIFT

(45) Veröffentlichungstag und Bekanntmachung des Hinweises auf die Patenterteilung:
**12.06.1996 Patentblatt 1996/24**

(21) Anmeldenummer: **91903368.8**

(22) Anmeldetag: **14.02.1991**

(51) Int Cl.[6]: **C07C 233/23**, A61K 31/16, C07C 235/40, C07D 295/185

(86) Internationale Anmeldenummer:
**PCT/EP91/00290**

(87) Internationale Veröffentlichungsnummer:
**WO 91/12230 (22.08.1991 Gazette 1991/19)**

(54) **SALPETERSÄUREESTER VON CYCLOHEXANOL-DERIVATEN**

NITRIC ACID ESTERS OF CYCLOHEXANOL DERIVATIVES

ESTERS D'ACIDE NITRIQUE DE DERIVES CYCLOHEXANOL

(84) Benannte Vertragsstaaten:
**AT BE CH DE DK ES FR GB GR IT LI LU NL SE**

(30) Priorität: **16.02.1990 DE 4004841**

(43) Veröffentlichungstag der Anmeldung:
**02.12.1992 Patentblatt 1992/49**

(73) Patentinhaber: **BOEHRINGER MANNHEIM GMBH
D-68298 Mannheim (DE)**

(72) Erfinder:
- **MICHEL, Helmut
D-6800 Mannheim 31 (DE)**
- **BARTSCH, Wolfgang
D-6806 Viernheim (DE)**

(56) Entgegenhaltungen:
**EP-A- 0 192 829** **EP-A- 0 367 019**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 515 420 B1

Printed by Jouve, 75001 PARIS (FR)

## Beschreibung

Die vorliegende Erfindung betrifft neue Salpetersäureester von Cyclohexanol-Derivaten der Formel I

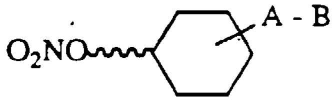

(I)

in der

| | |
|---|---|
| A | einen Valenzstrich oder eine $C_1$-$C_6$-Alkylenkette und |
| B | die Gruppe $-NR^1-CO-Z$, $-NR^1-SO_2-Z$ oder $-CO-NR^2-Z$ bedeuten, wobei |
| $R^1$ | Wasserstoff oder eine $C_1$-$C_6$-Alkylgruppe, |
| $R^2$ | Wasserstoff, eine Hydroxy-, Hydroxy-$C_1$-$C_6$-Alkyl-, $C_1$-$C_6$-Alkoxy-, $C_1$-$C_6$-Alkyl-, $C_2$-$C_6$-Alkenyl- oder $C_2$-$C_6$- Alkinylgruppe und |
| Z | Wasserstoff, eine $C_1$-$C_6$-Alkyl-, $C_2$-$C_6$-Alkenyl- oder $C_2$-$C_6$-Alkinylgruppe bedeutet, die ein- oder mehrfach durch eine Hydroxy-, $C_1$-$C_6$-Alkylcarbonyloxy-, $C_1$-$C_6$-Alkoxy-, Halogen-, Cyano-, Carboxy-, $C_1$-$C_6$-Alkoxycarbonyl-, $-CO-NR^3R^4$, Mercapto-$C_1$-$C_6$-Alkylmercapto- oder $C_1$-$C_6$-Alkylcarbonylmercapto-gruppe substituiert sein können, oder Z eine $C_3$-$C_6$-Cycloalkylgruppe oder einen Pyridin-, N-Oxypyddin-, Tetrazolyl- oder Pyrrolidinonring darstellen, oder Z zusammen mit $R^2$ und dem Stickstoffatom, an das Z und $R^2$ gebunden sind, einen heterocyclischen Ring bildet, der zusätzlich ein Sauerstoff-, Schwefel- oder Stickstoffatom enthalten kann, wobei der heterocyclische Ring mit einem zusätzlichen Stickstoffatom gegebenenfalls am Stickstoffatom durch eine $C_1$-$C_6$-Alkylcarbonylgruppe acyliert sein kann, und für den Fall, daß B eine $-NR^1-CO-Z$- Gruppe ist, Z auch eine $C_1$-$C_6$-Alkoxygruppe bedeuten kann, oder wenn Z eine durch eine Mercapto-, $C_1$-$C_6$-Alkylmercapto- oder $C_1$-$C_6$-Alkylcarbonyl-mercapto substituierte $C_1$-$C_6$-Alkyl- oder $C_2$-$C_6$-Alkenylgruppe bedeutet, die $C_1$-$C_6$-Alkyl- oder $C_2$-$C_6$-Alkenylgruppe zusätzlich durch die Gruppe $-NR^4R^5$ oder eine $C_1$-$C_4$-Alkylcarbonyl-gruppe substituiert sein kann, und |
| $R^3$ und $R^4$ | gleich oder verschieden sein können und jeweils unabhängig voneinander Wasserstoff, eine $C_1$-$C_6$-Alkylgruppe oder $R^3$ und $R^4$ zusammen mit dem Stickstoffatom, an das sie gebunden sind, einen heterocyclischen Ring bilden, der zusätzlich ein Sauerstoff-, Schwefel- oder Stickstoffatom enthalten kann, und |
| $R^5$ | Wasserstoff, Methyl oder Acetyl bedeutet, |

deren optisch aktive Formen und physiologisch verträgliche Salze, sowie Arzneimittel, die diese Verbindungen enthalten.

Ähnliche Verbindungen mit Nitroxyfunktion sind aus den früheren europäischen Patentanmeldungen EP-A-0,367,019 und EP-A-0,366,004 bekannt. EP-A-0,367,019 beschreibt u.a. Nitroxycyclohexylamine, die eine endständige basische Aminogruppe besitzen. In EP-A-0,366,004 sind Nitroxyverbindungen offenbart, bei denen die Nitroxygruppe über die Gruppe -A-B an einen Pyrrolidin- oder Piperidinring gebunden ist. In EP-A-0,359,335 werden ferner Arzneimittel beschrieben, die Nitratesterderivate als Wirkstoffe zur Behandlung von Herz- und Kreislauferkrankungen enthalten. Dort werden in den Beispielen 35-40 Cyclohexanoldinitrate offenbart, die in 2-, 3- und 4-Stellung des Cyclohexanringes substituiert sind, bzw. ein Cyclohexanolnitrat, das in 2-Stellung eine Hydroxygruppe besitzt.

Die erfindungsgemäßen Verbindungen der allgemeinen Formel I besitzen wertvolle pharmakologische Eigenschaften. Sie bewirken eine Verminderung des Sauerstoffbedarfs des Herzens, eine Erhöhung des Blutflusses und eine Erniedrigung des Blutdrucks. Überraschenderweise wurde nun gefunden, daß die beanspruchten Verbindungen eine nitratartige Wirkung von besonders langer Wirkdauer aufweisen. Sie eignen sich daher zur Prophylaxe und/oder Behandlung von Herz- und Kreislauferkrankungen, wie z.B. Angina pectoris.

Die Gruppe A-B kann in 2-, 3- oder 4-Stellung des Cyclohexylrings gebunden sein. wobei die 3- und 4-Stellung besonders bevorzugt ist. Die Gruppe A-B kann cis- oder trans-ständig zur Nitroxygruppe stehen, wobei die trans-Stellung bevorzugt ist.

Die "Alkyl-", "Alkenyl-" oder "Alkinyl-"teile bei den zuvor genannten Gruppen, wie z.B. die Alkyl-, Alkoxy- oder Alkylcarbonyloxygruppe, können in allen Fällen geradkettig oder verzweigt sein und 1-6 bzw. 2-6 Kohlenstoffatome enthalten, bevorzugt 1-4 bzw. 2- 4 Kohlenstoffatome. Geradkettige Alkylteile sind beispielsweise der Methyl-, Ethyl-, n-Propyl-, n-Butyl- oder n-Pentylrest. Verzweigtkettige Alkylteile sind beispielsweise die Gruppen $-CH(CH_3)-$, $-C(CH_3)_2-$, $-CH(CH_3)-CH_2-$, $-C(CH_3)_2-CH_2-$ oder $-CH_2-CH(CH_3)-CH_2-$. Die "Alkenyl"-teile sind vor allem geradkettige Reste, wie z.B. die Vinyl-, 1-Propenyl- oder 2-Propenylgruppe. Die "Alkinyl"-teile sind geradkettig oder verzweigt, beispielsweise die Propargyl- oder 2-Methyl-3-butinylgruppe.

In der Formel I kann A einen Valenzstrich oder eine geradkettige oder verzweigte $C_1$-$C_6$-Alkylengruppe bedeuten, wobei die Methylen-, Ethylen-, Methylmethylen- und Dimethylmethylengruppe bevorzugt sind.

$R^1$ kann Wasserstoff oder eine $C_1$-$C_6$-Alkylgruppe beispielsweise die Methyl-, Ethyl-, n-Propyl- und Isopropylgruppe bedeuten.

$R^2$ kann Wasserstoff, eine Hydroxy- oder Alkoxygruppe von 1-6 C- Atomen bedeuten. Bevorzugt ist die Methoxy-, Ethoxy-, n-Propoxy- und Isopropoxygruppe. Bedeutet R2 eine geradkettige oder verzweigte Alkyl- oder Hydroxyalkylgruppe von 1-6 C-Atomen, so kommt die Methyl-, Ethyl-, n-Propyl-, Isopropyl-, n-Butyl-, Isobutyl-, n- Pentyl-, Isopentyl-, n-Hexyl- und Isohexylgruppe in Frage. $R^2$ kann auch eine geradkettige oder verzweigte $C_2$-$C_6$-Alkenyl- oder Alkinylgruppe bedeuten. Bevorzugt sind die Vinyl-, Allyl-, 2- Methylallyl- und die 2-Methyl-3-butinylgruppe. Für den Fall, daß $R^2$ gemeinsam mit Z einen Ring bildet, der gegebenenfalls durch ein Sauerstoff-, Schwefel- oder gegebenenfalls acyliertes Stickstoffatom unterbrochen sein kann, kommen der Aziridin-, der Azetidin-, der Pyrrolidin-, der Piperidin-, der Morpholin-, der Thiomorpholin und der N-Acetylpiperazin-Ring in Frage.

Z kann Wasserstoff, eine geradkettige oder verzweigte $C_1$-$C_6$-Alkylgruppe oder eine geradkettige oder verzweigte $C_2$-$C_6$-Alkenyl- oder Alkinylgruppe bedeuten. Diese Gruppen können ein-, zwei- oder dreifach substituiert sein, wobei die Substituenten gleich oder verschieden sein können. Alkyl-, Alkenyl- oder Alkinylgruppen, die bevorzugt ein- oder zweifach substituiert sein können, sind insbesondere solche Gruppen mit bis zu 5 C-Atomen, wie z.B. die Methyl-, Ethyl-, n-Propyl-, i-Propyl-, 2-Methyl-2-propyl-, t-Butyl-, Vinyl-, Propargyl- oder 2-Methyl-3-butinylgruppe. Die in der Definition von Z genannten Substituenten dieser Gruppen können an jedem beliebigen C-Atom stehen. Bevorzgte Reste für Z sind die folgenden: $-CH_2-$, $-CH(CH_3)-$, $-C(CH_3)_2-$, $-CH_2-CH_2-$, $-CH(CH_3)-CH_2-$, $-C(CH_3)_2-CH_2-$, $-CH_2-CH_2-CH_2-$, $-CH_2-CH(CH_3)-CH_2-$ und $-CH=CH-$. Als Substituenten dieser Reste kommen insbesondere die folgenden in Frage: ein Halogenatom, wie z.B. ein Chlor- oder Bromatom, eine Carboxy-, $R^3R^4N$-CO-, $C_1$-$C_6$-Alkoxy-, wie z.B. Methoxy- oder Ethoxy-; $C_1$-$C_6$-Alkylcarbonyloxy-, wie z.B. Methylcarbonyloxy-; Hydroxy-; Cyano- oder $C_1$-$C_6$-Alkylcarbonylmercaptogruppe, wobei $R^3$ und $R^4$ gleich oder verschieden sein können und ein Wasserstoffatom oder eine $C_1$-$C_6$-Alkylgruppe darstellen. Bevorzugte Reste, die zwei Substituenten tragen, sind $C_1$-$C_6$-Alkylgruppen, insbesondere der $-C(CH_3)(CH_2-)(CH_2-)$-Rest, wobei als Substituenten bevorzugt die Hydroxy- oder Alkylcarbonyloxygruppe in Frage kommen. Beispielhaft genannt seien die Gruppen $-C(CH_3)(CH_2OH)_2$ und $-C(CH_3)(CH_2-O-CO-CH_3)_2$.

Z ist vorzugsweise die Methyl-, Ethyl-, n-Propyl-, Isopropyl-, n-Butyl-, tert.-Butyl-, Vinyl-, Allyl-, 2-Methylallyl-, die Hydroxymethyl-, Acetoxymethyl-, Methoxymethyl-, Ethoxymethyl-, Isopropoxymethyl-, Brommethyl-, Chlormethyl- oder die Cyanomethyl-gruppe, sowie die 1-(Hydroxy-)ethyl-, 1-(Acetoxy)ethyl-, 1-(Methoxy)ethyl., 1-(Ethoxy)ethyl-, 1-(Isopropoxy)ethyl-, die Mercaptomethyl-, Methylmercaptomethyl-, Acetylmercaptomethyl-, 1-Mercaptoethyl-, 1-(Methylmercapto)-ethyl-, 1-(Acetylmercapto)-ethyl-, 2-Mercaptoethyl-, 2-(Methylmercapto)ethyl-, 2-(Acetyl-mercapto) ethyl-, 1-Ethoxycarbonyl-2-mercapto-ethyl-, 1-Ethoxy-carbonyl-2-methylmercaptoethyl-, 1-Ethoxycarbonyl-2-acetylmercapto-ethyl-, 1-Ethoxycarbonyl-3-mercapto-propyl-, 1-Ethoxy-carbonyl-3-methylmercapto-propyl-, 1-Ethoxycarbonyl-3-acetyl-mercapto-propyl-gruppe, sowie die 2-(Hydroxy)ethyl-, 3-(Hydroxy)propyl- oder die 1,1-Dimethyl-2-hydroxyethylgruppe.

Weiterhin kommen für Z die Hydroxycarbonylmethyl-, 1-(Hydroxycarbonyl)ethyl-, 2-(Hydroxycarbonyl)ethyl-, 3-(Hydroxycarbonyl)-propyl-, 2-(Hydroxycarbonyl)propyl-, 2-(Hydroxy-carbonyl)vinyl-, Methoxycarbonylmethyl-, Ethoxycarbonylmethyl-, 1-(Methoxycarbonyl)-ethyl-, 1-(Ethoxycarbonyl)ethyl-, 2-(Methoxycarbonyl)ethyl-, 2-(Ethoxycarbonyl)ethyl-, 3-(Methoxycarbonyl)propyl-, 3-(Ethoxy-carbonyl)propyl-, 2-(Methoxycarbonyl)propyl-, 2-(Ethoxycarbonyl)-propyl-, 2-(Methoxycarbonyl)vinyl-, 2-(Ethoxycarbonyl)vinyl-, Aminocarbonylmethyl-, 1-(Aminocarbonyl)ethyl-, 2-(Aminocarbonyl)ethyl-, 2-(Aminocarbonyl)propyl-, 3-(Aminocarbonyl)propyl-, 2-(Aminocarbonyl)-vinyl-, Methylaminocarbonylmethyl-, 1-(Methylaminocarbonyl)ethyl-, 2-(Methylaminocarbonyl)ethyl-, 2-(Methylaminocarbonyl)propyl-, 3-(Methylaminocarbonyl)propyl-, 2-(Methylaminocarbonyl)vinyl-, Dimethylaminocarbonylmethyl-, 1-(Dimethylaminocarbonyl)ethyl-, 2-(Dimethylaminocarbonyl)ethyl-, 2-(Dimethyl-aminocarbonyl)propyl-, 3-(Dimethylaminocarbonyl)propyl-, 2-(Dimethylaminocarbonyl)vinylgruppe in Frage.

Für den Fall, daß Z eine Alkyl- oder Alkenylgruppe ist, die durch die Gruppe $-CO-NR^3R^4$-substituiert ist und $R^3$ und $R^4$ einen Ring bilden können, der gegebenenfalls noch durch ein Sauerstoff-, Schwefel- oder acyliertes Stickstoffatom unterbrochen sein kann, kommen die Pyrrolidinocarbonylmethyl-, Piperidinocarbonylmethyl-, Morpholinocarbonylmethyl-, Thiomorpholinocarbonylmethyl-, die N- Acetylpiperazinocarbonylmethyl-, 1-(Pyrrolidinocarbonyl)ethyl-, 1-(Piperidinocarbonyl)ethyl-, 1-(Morpholinocarbonyl)ethyl-, 1-(Thiomorpholinocarbonyl)ethyl-, 1-[(N-Acetyl)piperazinocarbonyl]ethyl-, 2-(Pyrrolidinocarbonyl)-ethyl-, 2-(Piperidinocarbonyl)ethyl-, 2-(Morpholinocarbonyl)ethyl-, 2-(Thiomorpholinocarbonyl)ethyl-, 2-[(N-Acetyl)piperazino-carbonyl]ethyl-, 3-(Pyrrolidinocarbonyl)propyl-, 3-(Piperidino)-3-carbonyl)propyl-, 3-(Morpholino-carbonyl)propyl-, 3-(Thiomorpholinocarbonyl)propyl-, 3-[(N-Acetyl)piperazinocarbonyl]-propyl-, 2-(Pyrrolidinocarbonyl)propyl-, 2-(Piperidinocarbonyl)-propyl-, 2-(Morpholinocarbonyl)propyl-, 2-(Thiomorpholinocarbonyl)-propyl-, 2-[(N-Acetyl)piperazinocarbonyl]propyl-, 2-(Pyrrolidinocarbonyl)vinyl-, 2-(Piperidinocarbonyl) vinyl-, 2-(Morpholino-carbonyl)vinyl-, 2-(Thiomorpholinocarbonyl)-vinyl- und die 2-[(N- Acetyl)piperazinocarbonyl] vinylgruppe in Frage.

Für den Fall, daß Z eine durch eine Mercapto-, $C_1$-$C_6$-Alkylmercapto- oder $C_1$-$C_6$-Alkylcarbonylmercapto substituierte $C_1$-$C_6$-Alkyl- oder $C_2$-$C_6$-Alkenylgruppe darstellt, kann diese zusätzlich noch durch eine Amino-, $C_1$-$C_3$-Alkylamino- oder $C_1$-$C_3$-Alkylcarbonylaminogruppe substituiert sein. Bevorzugt in diesem Sinne sind die 1-Amino-2-mercaptoethyl-, 1-Amino-2-methylmercapto-ethyl-, 1-Amino-2-acetylmercapto-ethyl-, 1-Methylamino-2-mercapto-ethyl-, 1-Methylamino-2-methylmercapto-ethyl-, 1-Methylamino-2-acetylmercapto-ethyl-, 1-Acetyl-amino-2-mercapto-ethyl-, 1-Acetylamino-methyl-mercapto-ethyl-, 1 -Acetyl-amino-2-acetylmercapto-ethyl-, 1-Amino-2-mercapto-2-methyl-propyl-, 2-Amino-3-mercapto-propyl-, 1-Amino-3-methylmercapto-propyl-, 1-Amino-3-acetylmercapto-propyl-, 1-Methylamino-3-mercapto-propyl-, 1-Methylamino-3-methylmercapto--propyl-, 1-Methylamino-3-acetylmercapto-propyl-, 1-Acetylamino-3-mercapto-propyl-, 1-Acetylamino-3-methylmercapto-propyl-, 1-Acetylamino-3-acetyl-mercapto-propyl-gruppe.

Für den Fall, daß Z einen $C_3$-$C_6$-Cycloalkylrest darstellt, versteht man darunter den Cyclopropyl-, Cyclobutyl-, Cyclopentyl- oder Cyclohexylrest.

Bedeutet Z einen Pyridylrest oder dessen N-Oxid, kann die Verknüpfung in 2-, 3-oder 4-Position sein. Bedeutet Z den Tetrazol-5-yl-Rest, so ist B insbesondere die Gruppe -CO-$NR^2$-Z.

Für den Fall, daß $R^3$ und $R^4$ oder Z und $R^2$ zusammen einen heterocyclischen Ring mit einem Stickstoffatom bilden, der zusätzlich ein Sauerstoff-, Schwefel- oder Stickstoffatom enthalten kann, so versteht man darunter insbesondere einen Pyrrrolidin-, Piperidin-, Morpholin-, Thiomorpholin- oder Piperazinring oder deren N-cyclisierten Derivate, wie z.B. N-$C_1$-$C_6$-Alkylcarbonyl-Derivate.

Bevorzugt kommen die folgenden Bedeutungen für A und B in Formel I in Frage:

| | |
|---|---|
| A | bedeutet einen Valenzstrich oder eine $C_1$-$C_3$-Alkylengruppe, insbesondere die -$CH_2$-Gruppe. |
| B | bedeutet die Gruppen -$NR^1$-CO-Z, -$NR^1$-$SO_2$-Z oder -CO-$NR^2$-Z, wobei |
| $R^1$ | ein Wasserstoffatom oder eine $C_1$-$C_3$-Alkylgruppe, insbesondere die Methyl- oder Ethylgruppe darstellt, und |
| $R^2$ | ein Wasserstoffatom, eine Hydroxy-, Hydroxy-$C_1$-$C_3$-alkyl- oder $C_1$-$C_3$-Alkylgruppe, insbesondere die Methyl- oder Ethylgruppe, darstellt oder $R^2$ und Z zusammen eine Morpholinogruppe bilden, |
| $R^3$ und $R^4$ | unabhängig voneinander ein Wasserstoffatom oder eine $C_1$-$C_3$-Alkylgruppe, insbesondere die Methylgruppe, darstellen, |
| Z | ein Wasserstoffatom, eine $C_1$-$C_6$-Alkylgruppe, wie z.B. die Methyl-, Ethyl-, tert.-Butyl- oder n-Pentylgruppe; eine $C_2$-$C_6$-Alkenylgruppe, wie z.B. die Vinylgruppe; oder eine $C_2$-$C_6$-Alkinylgruppe, wie z.B. -C$(CH_3)_2$-C≡CH, wobei die Alkyl- und Alkenylgruppen einfach substituiert sein können durch ein Halogenatom, wie z.B. Chlor- oder Bromatom; eine Carboxy-; Carboxamido-, $C_1$-$C_3$-Alkoxy-, $C_1$-$C_3$-Alkylcarbonyloxy-, Hydroxy- oder Cyanogruppe; wie z.B. die Gruppen -$CH_2Cl$, -$C(CH_3)_2$-Br, -$CH_2$-COOH, -CH$(CH_3)$-COOH, -$CH_2CH_2COOH$, -CH=CH-COOH, -$CH_2$-$CONH_2$, -$C(CH_3)_2$-$CONH_2$, -$CH_2$-$CH_2$-$CONH_2$, -CH=CH-$CONH_2$, -$CH_2$-CO-$N(CH_3)_2$, -$CH_2$-$CH_2$-CON $(CH_3)_2$, -$CH_2$-$OCH_3$, -$CH_2$-O-$COCH_3$, -CH$(CH_2)$-O-$COCH_3$, -$C(CH_3)_2$-$CH_2$-O-$COCH_3$, -$CH_2OH$, -$CH_2CH_2OH$, -$CH_2CH_2CH_2OH$, -CHOH-$CH_3$, -$C(CH_3)_2$-CH,OH, -$CH_2$-CH$(CH_3)$-$CH_2OH$, oder die Alkyl- und Alkenylgruppen zweifach substituiert sein können durch Hydroxy- oder $C_1$-$C_3$-Alkylcarbonyloxygruppen, wie z.B. -C$(CH_3)(CH_2OH)_2$ oder -CH$(CH_3)(CH_2$-O-$COCH_3)_2$. Z bedeutet ferner eine 3- oder 4-Pyridylgruppe bzw. N-Oxy-pyridylgruppe oder den Pyrrolidinonring. Für den Fall, daß B eine -$NR^2$-CO-Z-Gruppe bedeutet, ist Z bevorzugt eine $C_1$-$C_3$-Alkoxygruppe, wie z.B. die Ethoxygruppe. |

Weiterhin kommen solche Cyclohexanolnitrate der Formel I bevorzugt in Frage, in der

| | |
|---|---|
| A | einen Valenzstrich oder eine $C_1$-$C_6$-Alkylenkette und |
| B | die Gruppe -$NR^1$-CO-Z oder -CO-$NR^2$-Z bedeuten, wobei $R^1$ Wasserstoff oder eine $C_1$-$C_6$-Alkylgruppe, $R^2$ Wasserstoff, eine Hydroxy-, $C_1$-$C_6$-Alkoxy-, $C_1$-$C_6$-Alkyl-, $C_2$-$C_6$-Alkenyl- oder $C_2$-$C_6$-Alkinylgruppe und |
| Z | ein Wasserstoff, eine $C_1$-$C_6$-Alkyl- oder $C_2$-$C_6$-Alkenylgruppe, die ein- oder mehrfach durch eine Hydroxy-, $C_1$-$C_6$-Alkylcarbonyloxy-, $C_1$-$C_6$-Alkoxy-, Halogen-, Cyano-, Carboxy-, $C_1$-$C_6$-Alkoxycarbonyl- oder -CO-$NR^3R^4$-Gruppe substituiert sein können, oder Z eine $C_3$-$C_6$-Cycloalkylgruppe oder einen Pyridin-, N-Oxypyridin-, Tetrazolyl- oder Pyrrolidinonring, darstellen, oder Z zusammen mit $R^2$ einen heterocyclischen Ring bildet, der zusätzlich ein Sauerstoff-, Schwefel- oder Stickstoffatom enthalten kann, und für den Fall, daß B die Gruppe -$NR^1$-CO-Z darstellt, Z auch eine $C_1$-$C_6$-Alkoxygruppe bedeuten kann, |
| $R^3$ und $R^4$ | gleich oder verschieden sein können und jeweils unabhängig voneinander Wasserstoff, eine $C_1$-$C_6$-Alkulgruppe oder $R^3$ und $R^4$ zusammen mit dem Stickstnffatom, an das sie gebunden sind, einen heterocyclischen Ring bilden, der zusätzlich ein Sauerstoff-, Schwefel- oder Stickstoffatom enthalten kann, |

sowie deren optisch aktive Formen und physiologisch verträgliche Salze.

Dabei sind folgende Bedeutungen bevorzugt:

A bedeutet einen Valenzstrich oder eine $C_1$-$C_3$-Alkylengruppe,
$R^1$ bedeutet ein Wasserstoffatom oder die Methyl- oder Ethylgruppe,
$R^2$ bedeutet ein Wasserstoffatom oder ein Hydroxy- oder $C_1$-$C_4$-Alkylgruppe bedeutet,
$R^3$ und $R^4$ sind gleich oder verschieden und bedeuten ein Wasserstoffatom oder eine $C_1$-$C_3$-Alkylgruppe, oder $R^3$ und $R^4$ bzw. Z und $R^2$ zusammen mit dem Stickstoffatom, an das sie gebunden sind, bilden einen Pyrrolidin-, Piperidin-, Morpholin-, Thiomorpholin-oder Piperazinring, sowie deren N-acylierten Derivate,
Z bedeutet ein Wasserstoffatom, eine $C_1$-$C_6$-Alkyl- oder $C_2$-$C_6$-Alkenylgruppe, die durch eine Hydroxy-, Carboxy-, Halogen-, Cyano- oder -CO-$NR^3R^4$-Gruppe substituiert sein können, oder Z ist eine Pyrrolidin-2-on- oder Pyridinylgruppe.

Die erfindungsgemäßen Verbindungen der allgemeinen Formel I, können in an sich bekannter Weise dadurch hergestellt werden, indem man

1) eine Verbindung der allgemeinen Formel II,

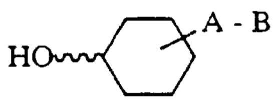

(II)

in der A und B die oben angegebenen Bedeutungen haben, einer Nitratester-Bildungsreaktion unterwirft,

2) oder eine Verbindung der allgemeinen Formel III,

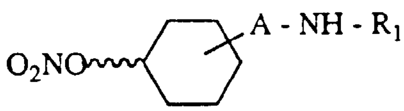

(III)

mit einer Verbindung der allgemeinen Formel IV,

W-CO-Z (IVa)

oder

$W-SO_2-Z$ (IVb)

wobei $R^1$ und Z die oben angegebenen Bedeutungen haben und W eine reaktionsfähige Gruppe darstellt umsetzt, oder

3) eine Verbindung der allgemeinen Formel V,

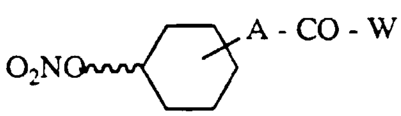

(V)

mit einer Verbindung der allgemeinen Formel VI,

$R^2$-NH-Z (VI)

wobei $R^2$, W, A und Z die angegebenen Bedeutungen haben, umsetzt

und gegebenenfalls anschließend so hergestellte Verbindungen der Formel I nachträglich in andere Verbindungen der Formel I umwandelt.

Einige Verbindungen der allgemeinen Formel II sind neu. Sie können hergestellt werden, indem man in an sich bekannter Weise

1) eine Verbindung der allgemeinen Formel VII,

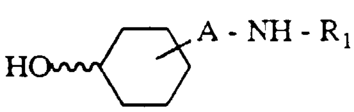

(VII)

wobei $R^1$ und A die oben angegebenen Bedeutungen haben, mit einer Verbindung der allgemeinen Formel IV,

umsetzt, oder

2) eine Verbindung der allgemeinen Formel VIII

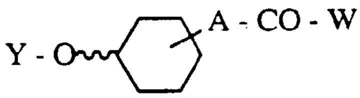

(VIII)

wobei A und W die angegebenen Bedeutungen haben und Y eine Schutzgruppe darstellt, mit einer Verbindung der allgemeinen Formel VI umsetzt und anschließend die Schutzgruppe Y abspaltet.

Die Nitratester-Bildungsreaktion zur Herstellung von Verbindungen der Formel I kann durchgeführt werden, indem man die Verbindungen der allgemeine Formel II mit einem salpetersäureesterbildenden Reagenz, wie rauchender Salpetersäure, einer Mischung aus rauchen- der Salpetersäure und Acetanhydrid, einer Mischung aus rauchender Salpetersäure und konz. Schwefelsäure oder Distickstoffpentoxid bei niedrigen Temperaturen in Gegenwart oder Abwesenheit eines inerten Lösungsmittel umsetzt.

Die Reaktionstemperaturen liegen zwischen Raumtemperatur und -60°C, bevorzugt zwischen -30°C und 0°C. Das Molverhältnis der Reaktionspartner liegt zwischen 1 und 10.

Als reaktionsfähige Gruppe W kommen Halogenide, wie Chlor oder Brom, Alkylcarboxylate oder die Hydroxylgruppe in Frage. Die ent- sprechenden aktivierten Carbonsäuren IV und V liegen daher in Form von Estern, Lactonen, Carbonsäurehalogeniden oder -anhydriden vor. Die Aktivierung der Carbonsäuren kann aber auch durch aktivierende Reagenzien wie N,N-Carbonyldiimidazol, N,N-Dicyclohexylcarbodiimid oder Chlorameisensäureester erfolgen. Die molaren Verhältnisse zwischen den Reaktionspartnern können zwischen 1 und 100 gewählt werden.

Als Schutzgruppe Y kommen die üblichen Hydroxyschutzgruppen in Frage, beispielsweise die Acetylgruppe.

Verbindungen der allgemeinen Formel VII, worin A einen Valenzstrich, $R^1$ Wasserstoff oder Acetyl bedeutet, werden in Ber. 72, 995, (1939) beschrieben. Ähnliche Verbindungen können auf analoge Weise hergestellt werden. Bedeutet A in Formel VII einen Valenzstrich und $R^1$ eine Chloracetyl- oder Chlorpropionylgruppe, so sind diese Verbindungen als Zwischenprodukte in EP-A-367,019 beschrieben. Andere Verbindungen der Formel VII können analog hergestellt werden.

Verbindungen der allgemeinen Formel VIII, worin A einen Valenzstrich, Y eine Acetylgruppe und W eine Hydroxylgruppe bedeutet, können wie in J. Chem. Soc. 1950, 1379 beschrieben, hergestellt werden. Andere Verbindungen der Formel VIII können analog hergestellt werden.

Die Verbindungen III und V sind aus EP-A-192,829 bekannt und werden dort als Zwischenprodukte der Formeln XI und IX beschrieben.

Nachträgliche Umwandlungen von Verbindungen der Formel I in andere Verbindungen der Formel I erfolgen beispielsweise dadurch, daß man eine Carboxylgruppe durch Umsetzung mit Alkoholen in die entsprechenden Esterderivate umwandelt oder durch Umsetzung mit Aminen in die entsprechenden Amide. Die eingesetzten Carbonsäuren können gegebenenfalls zuvor in die aktiven Carbonsäurederivate, wie z.B. Anhydride oder Halogenide nach an sich bekannten Methoden umgewandelt werden. Falls Z einen durch eine Alkylcarbonyloxygruppe substituierten Rest darstellt, so können solche Verbindungen durch Hydrolyse dieses Esters in die entsprechenden hydroxysubstituierten Derivate umgewandelt werden. Bedeutet Z einen N-Oxy-pyridinring, so werden diese bevorzugt aus den entsprechenden Pyridinderivaten der Formel I hergestellt, indem diese nachträglich durch sauerstoffgruppenübertragende Reagenzien, wie z.B. Wasserstoffperoxid, oxidiert werden.

Die erfindungsgemäßen Verbindungen der allgemeinen Formel I sind Cyclohexanderivate, deren Substituenten $-ONO_2$ und A-B in 1,2- 1,3 oder 1,4-Position sein können. Die Konfiguration kann jeweils cis oder trans sein. Für den Fall der 1,2- und 1,3-Position besitzen die erfindungsgemäßen Verbindungen zwei chirale Kohlenstoffatome. Gegenstand der Erfindung sind daher auch sämtliche Racemate, Diastereomerengemische und optisch aktive Formen der erfindungsgemäßen Verbindungen der allgemeinen Formel I.

Die Herstellung der pharmakologisch verträglichen Salze der Verbindungen der Formel I erfolgt durch Umsetzung mit Alkali- oder Erdalkalihydroxiden- bzw. -Carbonaten oder mit organischen Basen, wie z.B. Triethylamin.

Die Substanzen der allgemeinen Formeln I und ihre Salze können in flüssiger und fester Form enteral oder parenteral appliziert werden. Als Injektionsmedium, kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- oder Citratpuffer, Ethanol, Komplexbildner (wie Ethylendiamintetraessigsäure oder deren nicht toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole). Für orale Applikation geeignete Zubereitungen können gewünschtenfalls Geschmacks- und Süßstoffe enthalten.

Arzneimittel enthaltend Verbindungen der Formel I werden vorzugsweise einmal täglich oral verabreicht. Die Arzneimittel können die erfindungsgemäßen Verbindungen in einer Menge von 1 - 50 mg, vorzugsweise 5 - 30 mg pro Applikationsform enthalten.

Neben den nachfolgend aufgeführten Beispielen kommen auch die folgenden Verbindungen beispielhaft im Sinne der vorliegenden Erfindung in Frage:

1. N-Methyl-N-(trans-4-nitroxycyclohexyl)bernsteinsäuremonoamid
2. N-Methyl-N-(trans-4-nitroxycyclohexyl)bernsteinsäurediamid
3. N-Methyl-N-(trans-4-nitroxycyclohexyl)N',N'-dimethylbernstein-säurediamid
4. Cis-N-Formyl-3-nitroxycyclohexylamin
5. N-(cis-3-Nitroxycyclohexyl)bernsteinsäuremonoamid
6. N-(cis-3-Nitroxycyclohexyl)bernsteinsäurediamid
7. N-(cis-3-Nitroxycyclohexyl)N'N'-dimethylbemsteinsäurediamid
8. trans-N-Formyl-2-nitroxycyclohexylamin
9. trans-N-Acetyl-2-nitroxycyclohexylamin
10. N-(trans-2-Nitroxycyclohexyl)bernsteinsäuremonoamid
11. N- (trans-2-Nitroxycyclohexyl)bernsteinsäurediamid
12. N-(trans-2-Nitroxycyclohexyl)-N'N'-dimethylbernsteinsäurediamid
13. 2-Cyan-2-methyl-N-(trans-4-nitroxycyclohexyl)propionsäureamid
14. cis-N-Formyl-4-nitroxycyclohexylamin
15. trans-4-Nitroxycyclohexancarbonsäure-N-(tetrazol-5-yl)amid.
16. 2-Methylmercapto-N-(trans-4-nitroxycyclohexyl)essig-säureamid
17. 2-Amino-3-methylmercapto-N-(trans-4-nitroxycyclohexyl)propionsäureamid
18. 2-Methylamino-3-methylmercapto-N-(trans-4-nitroxycyclohexyl)propionsäureamid
19. 2-Methylamino-3-acetylmercapto-N-(trans-4-nitroxycyclohexyl)propionsäureamid
20. 2-Acetylamino-3-mercapto-N-(trans-4-nitroxycyclohexyl)propionsäureamid
21. 2-Acetylamino-3-acetylmercapto-N-(trans-4-nitroxycyclohexyl)propionsäureamid
22. 2-Amino-3-mercapto-3-methyl-(N-trans-4-nitroxycycloexyl)buttersäureamid
23. 2-Amino-4-methylmercapto-N-(trans-4-nitroxycyclohexyl)buttersäureamid
24. 2-Acetylamino-4-methylmercapto-N-(trans-4-nitroxycyclohexyl)buttersäureamid
25. trans-4-Nitroxycyclohexancarbonsäure-N-[2-(3-mercapto)propionsäureethylester]amid
26. trans-4-Nitroxycyclohexancarbonsäure-N-[2-(3-acetylmercapto)propionsäureethylester]amid
27. trans-4-Nitroxycyclohexancarbonsäure-N-[2-(4-methylmercapto)buttersäureethylester]amid
28. trans-4-Nitroxycyclohexancarbonsäure-N-(2-mercaptoethyl)amid
29. 2-Mercapto-N-(trans-4-nitroxycyclohexyl)essigsäureamid

Beispiel 1

trans-N-Acetyl-4-nitroxycyclohexylamin

28.3 ml (0.3 mol) Essigsäureanhydrid werden mit 375 ml Acetonnitril gemischt, im Eisbad auf 0-5°C abgekühlt und 12.6 ml (0.3 mol) 100proz. Salpetersäure zugetropft. Nach 30 Minuten werden unter Rühren und Kühlen bei 0-5°C 15.7 g (0.1 mol) trans-N-Acetyl-4-hydroxycyclohexylamin fest zugegeben. Die Reaktionsmischung wird noch 3 h bei 0-5°C nachgerührt und anschließend vorsichtig zu einer Lösung von 150 g (1.8 mol) Natriumhydrogen-carbonat in 500 ml Eiswasser zufließen lassen. Nach Extrahieren mit Essigester, Trocknen der organischen Phase mit Natriumsulfat und Abdestillieren im Vacuum verbleiben 17.8 g Rohprodukt. Durch Umkristallisieren aus Essigester werden 10.3 g der Titelverbindung vom Schmp. 146-148°C erhalten, d.s. 50 % d.Th..

In analoger Weise werden erhalten:

1/1: trans-N-n-Hexanoyl-4-cyclohexylamin
aus trans-N-n-Hexanoyl-4-hydroxycyclohexylamin
Schmelzpunkt: 100-101°C (Ether), Ausbeute: 58 %

1/2: trans-4-Nitroxycyclohexancarbonsäureamid
aus trans-4-Hydroxycyclohexancarbonsäureamid
Schmelzpunkt: 160-159°C (Essigester), Ausbeute: 62 %

1/3: trans-4-Nitroxvcyclohexancarbonsäuredimethylamid

aus trans-4-Hydroxycyclohexancarbonsäuredimethylamid
Schmelzpunkt: 108-110°C (Essigester), Ausbeute: 40 %

1/4: trans-4-Nitroxycyclohexancarbonsäuremorpholid
aus trans-4-Hydroxycyclohexancarbonsäuremorpholid
Schmelzpunkt: 89-90°C (Ether), Ausbeute: 40 %

1/5: cis-N-Acetvl-4-nitroxycyclohexylamin
aus cis-N-Acetyl-4-hydroxycyclohexylamin
Schmelzpunkt: 107-109°C (Ether), Ausbeute: 45 %

1/6: 2-Chlor-N-(trans-4-nitroxycyclohexyl)essigsäureamid
aus 2-Chlor-N-(trans-4-hydroxycyclohexyl)essigsäureamid
Schmelzpunkt: 102-104°C (Ether), Ausbeute: 34 %

1/7: 2-Brom-2-methyl-N-(trans-4-nitroxycyclohexyl)propion-säureamid
aus 2-Brom-2-methyl-N-(trans-4-hydroxycyclohexyl)-propion-säureamid
Schmelzpunkt: 92-94°C (Ether/Isohexan), Ausbeute: 60 %

1/8: N-(trans-4-Nitroxycyclohexyl)acrylsäureamid
aus N-(trans-4-Hydroxycyclohexyl)acrylsäureamid Schmelzpunkt: 160-162°C (Essigester), Ausbeute: 63 %

1/9: 2-Cyan-N-(trans-4-nitroxycyclohexyl)essigsäureamid
aus 2-Cyan-N-(trans-4-hydroxycyclohexyl)essigsäureamid
Schmelzpunkt: 149-150°C (Ether), Ausbeute 53 % d.Th.

Beispiel 2

trans-N-Acetyl-4-nitroxycyclohexylamin

Eine Lösung von 2.3 g (0.015 mol) trans-4-Nitroxycyclohexylamin in 25 ml Essigester wird mit 10 ml (0.13 mol) Essigsäure- anhydrid versetzt und über Nacht bei Raumtemperatur gerührt. Man kühlt auf 5°C ab, gibt 100 ml Ethanol zu und nach Stehen über Nacht bei Raumtemperatur wird im Vacuum abdestilliert. Nach Anreiben mit Ether werden die Kristalle abgesaugt. Es verbleiben 1.5 g der Titelverbindung vom Schmp. 146-148°C, d.S. 50 % d.Th..

Analog werden erhalten:

2/1: trans-N-Acetyl-4-nitroxycyclohexylmethylamin
aus trans-4-Nitroxycyclohexylmethylamin
Schmelzpunkt: 91-93°C (Ether), Ausbeute: 30 %

2/2: cis-N-Acetyl-3-nitroxycyclohexylamin
aus cis-3-Nitroxycyclohexylamin
Schmelzpunkt: 112-113°C (Ether), Ausbeute: 41 %

2/3: trans-N-Methyl-N-acetyl-4-nitroxycyclohexylamin
aus trans-N-Methyl-4-nitroxycyclohexylamin
Schmelzpunkt: 37-39°C (Ether/Isohexan), Ausbeute 77 % d.Th.

2/4: trans-N-Ethyl-N-acetyl-4-nitroxycyclohexylamin aus
trans-N-Ethyl-4-nitroxycyclohexylamin
Schmelzpunkt: 68-69°C (Ether), Ausbeute: 35 % d.Th.

Beispiel 3

trans-N-Formyl-4-nitroxycyclohexylamin

Eine Mischung aus 6.8 ml (0.072 mol) Essigsäureanhydrid und 2.8 ml (0.072 mol) Ameisensäure wird 2 h auf 60°C erwärmt und danach unter Kühlen bei 5°C mit 1.9 g (0.012 mol) trans-4-Nitroxycyclohexylamin versetzt. Man läßt über

Nacht bei Raumtemperatur rühren, verdünnt anschließend mit 100 ml Essigester und gibt 100 ml Wasser zu. Nach Neutralisieren mit gesättigter Natriumhydrogencarbonatlösung wird die organische Phase mit Natriumsulfat getrocknet und im Vacuum abdestilliert Nach Anreiben mit Ether werden die Kristalle abgesaugt. Man erhält 1.5 g der Titelverbindung vom Schmp. 148-150°C, d.s. 66 % d.Th..

Analog wird erhalten:

3/1: trans-N-Formyl-4-nitroxycyclohexylmethylamin
aus trans-4-Nitroxycyclohexylmethylamin
Schmelzpunkt: Öl, Ausbeute: 70 %

3/2: trans-N-Methyl-N-formyl-4-nitroxycyclohexylamin aus
trans-N-Methyl-4-nitroxycyclohexylamin
Schmelzpunkt: 52-54°C (Ether/Isohexan), Ausbeute 60 % d.Th.

Beispiel 4

N-(trans-4-Nitroxycyclohexyl)bernsteinsäure-monoamid

Zu einer Lösung von 4.8 g (0.03 mol) trans-4-Nitroxycyclohexyl-amin in 25 ml Acetonitril gibt man bei 10°C 3 g (0.03 mol) Bernsteinsäureanhydrid und läßt 48 h bei Raumtemperatur rühren. Nach Absaugen verbleiben 3.6 g der Titelverbindung vom Schmp. 136-138°C, d.s. 46 % d.Th..

Analog wird erhalten:

4/1: N-(trans-4-Nitroxycyclohexyl)maleinsäure-monoamid
aus trans-4-Nitroxycyclohexylamin und Maleinsäureanhydrid
Schmelzpunkt: 178-179°C (Acetonitril), Ausbeute: 47 %

4/2: N-(trans-4-Nitroxycyclohexylmethyl)bernsteinsäure-monoamid
aus trans-4-Nitroxycyclohexylmethylamin und Bernsteinsäureanhydrid
Schmelzpunkt: 79-81°C (Ether), Ausbeute: 80 %

Beispiel 5

N-(trans-4-Nitroxycyclohexyl)bernsteinsäurediamid

Eine Suspension von 3.9 g (0.015 mol) N-(trans-4-Nitroxycyclohexyl)bernsteinsäuremonoamid (Beispiel 4), in 100 ml abs. Methylenchlorid werden mit 3.1 g (0.015 mol) Phosphorpentachlorid unter Kühlen bei 5-10°C versetzt. Nach 2 h Rühren bei Raumtemperatur wird im Vacuum bei max. 20°C abdestilliert, mit abs. Ether verrieben und abgesaugt. Das Säurechlorid wird sofort in 50 ml 10proz. Ammoniak bei 5-10°C fest eingetragen und über Nacht bei Raumtemperatur gerührt. Nach Absaugen verbleiben 1.2 g der Titelverbindung vom Schmp. 167-169°C, d.s. 30 % d.Th..

Analog werden erhalten:

5/1: N-(trans-4-Nitroxycyclohexyl)maleinsäurediamid
aus N-(trans-4-Nitroxycyclohexyl)maleinsäuremonoamid (Beisp. 4/1) und Ammoniak
Schmelzpunkt: 151-152 °C (Essigester), Ausbeute: 31 %

5/2: N-(trans-4-Nitroxycyclohexyl)N'N'-dimethylbemstein-säurediamid
aus N-(trans-4 Nitroxycyclohexyl)bernsteinsäuremonoamid (Beisp. 4) und Dimethylamin
Schmelzpunkt: 120-122°C (Ether), Ausbeute: 42 %

5/3: N-(trans-4-Nitroxycyclohexylmethyl)bernsteinsäure-diamid
aus N-(trans-4-Nitroxycyclohexylmethyl)bernsteinsäuremonoamid und Ammoniak
Schmelzpunkt: 156-157°C (Essigester), Ausbeute: 63 %

5/4: N-(trans-4-Nitroxycyclohexylmethyl)-N',N'-dimethyl-bernsteinsäurediamid
aus N-(trans-4-Nitroxycyclohexylmethyl)-bernsteinsäuremonoamid und Dimethylamin
Schmelzpunkt: 70-71°C (Ether), Ausbeute: 40 %

Beispiel 6

trans-N-Propionyl-4-nitroxycyclohexylamin

Zu einer Lösung von 3.2 g (0.02 mol) trans-4-Nitroxycyclohexylamin in 80 ml abs. Methylenchlorid werden bei 5-10°C 3 ml (0.02 mol) Triethylamin zugegeben und anschließend 2 g (0.02 mol) Propionylchlorid in 20 ml abs. Methylenchlorid zugetropft. Man läßt über Nacht bei Raumtemperatur rühren, schüttelt 3 x mit 25 ml Wasser aus, trocknet die organische Phase mit Natriumsulfat und destilliert im Vacuum ab. Der Rückstand wird mit Ether verrieben und abgesaugt. Es verbleiben 2.8 g der Titel- verbindung vom Schmp. 138-140°C, d.s. 65 % d.Th..

Analog werden erhalten:

6/1: 2-Methoxy-N-(trans-4-nitroxycyclohexyl)essigsäureamid
aus trans-4-Nitroxycyclohexylamin und Methoxyessig-säurechlorid
Schmelzpunkt: 98-99 °C (Ether), Ausbeute: 40 %

6/2: 2-Acetoxy-N-(trans-4-nitroxycyclohexyl)essigsäureamid
aus trans-4-Nitroxycyclohexylamin und Acetoxy-essigsäurechlorid Schmelzpunkt: 98-99°C (Ether), Ausbeute: 90 %

6/3: 2-Acetoxy-N-(trans-4-nitroxycyclohexyl)pronionsäureamid
aus trans-4-Nitroxycyclohexylamin und 2-Acetoxy-propionsäurechlorid
Schmelzpunkt: 104-105°C (Essigester/Ether), Ausbeute: 80 %

6/4: 3-Acetoxy-N-(trans-4-nitroxycyclohexyl)2,2-dimethyl-propionsäureamid
aus trans-4-Nitroxycyclohexylamin und 3-Acetoxy-2,2-dimethyl-propionsäurechlorid
Schmelzpunkt: Öl, Ausbeute: 80 %

6/5: N-(trans-4-nitroxycyclohexyl)nicotinsäureamid
aus trans-4-Nitroxycyclohexylamin und Nicotinsäureazid
Schmelzpunkt: 171-173°C (Essigester), Ausbeute: 46 %

6/6: N-(trans-4-nitroxycyclohexyl)isonicotinsäureamid
aus trans4-Nitroxycyclohexylamin und Isonicotinsäureazid
Schmelzpunkt: 170-172°C (2-Propanol), Ausbeute: 40 %

6/7: 2-Acetoxy-N-(trans-4-nitroxycyclohexylmethyl)essig-säureamid
aus trans-4-Nitroxycyclohexyl-methylamin und Acetoxy-essigsäurechlorid
Schmelzpunkt: 65-66°C (Ether), Ausbeute: 60 %

6/8: N-trans-4-Nitroxycyclohexyl-2.2-dimethyl-propionsäureamid
aus trans-4-Nitroxycyclohexylamin und 2,2-Dimethyl-propionsäurechlorid
Schmelzpunkt: 148-151°C (Ether), Ausbeute 25 % d.Th.

6/9: N-trans-4-Nitroxycyclohexyl-methansulfonsäureamid aus
trans-4-Nitroxycyclohexylmin und Methansulfonsäurechlorid
Schmelzpunkt: 154-155°C (Ether), Ausbeute 84 % d.Th.

6/10: 2,2-Bis-(acetoxymethyl)-N-trans-4-nitroxycyclohexyl-pronionsäureamid
aus trans-4-Nitroxycyclohexylamin und
2,2-Bis-(acetoxymethyl)propionsäurechlorid
Schmelzpunkt: 95-97°C (Essigester), Ausbeute 40 % d.Th.

6/11: N-Ethoxycarbonyl-trans-4-nitroxycyclohexylamin aus
trans-4-Nitroxycyclohexylamin und Chlorameisen-säureethylester
Schmelzpunkt: 93-94°C (Ether/Isohexan), Ausbeute: 56 % d.Th.

6/12: 2-Acetylmercapto-N-(trans-4-nitroxycyclohexyl)-essig-säureamid
aus trans-4-Nitroxycyclohexylamin und 2-Acetylmercaptoessigsäurechlorid

Schmelzpunkt: 127-129°C (Ether), Ausbeute 67 % d.Th.

Beispiel 7

trans-4-Nitroxycyclohexancarbonsäure-N-(2-methyl-3-butin-2-yl)amid

6.2 g, (0.075 mol) 2-Methyl-3-butin-2-ylamin werden in 100 ml abs. Essigester gelöst und unter Kühlen bei 5-10°C 5.2 g (0.025 mol) trans-4-Nitroxycyclohexancarbonsäurechlorid in 25 ml abs. Essigester zugetropft. Nach 3 h Rühren bei Raumtemperatur wird mit 100 ml Wasser ausgeschüttelt, die Essigesterphase mit Natriumsulfat getrocknet und im Vacuum abdestilliert. Der Rückstand wird mit Ether versetzt und abgesaugt. Es verbleiben 2.6 g der Titelverbindung vom Schmp. 138-139°C, d.s. 40 % d.Th..

Analog werden erhalten:

7/1: trans-4-Nitroxycyclohexancarbonsäure-N-(2-hydroxyethvl)-amid
aus trans-4-nitroxycyclohexancarbonsäurechlorid und 2-Hydroxyethylamin
Schmelzpunkt: 132-133oC (Ether), Ausbeute: 25 %

7/2: trans-4-Nitroxycyclohexancarbonsäure-N-(3-hydroxy-propyl)amid
aus trans-4-nitroxycyclohexancarbonsäurechlorid und 3-Hydroxypropylamin
Schmelzpunkt: 78-79oC (Ether), Ausbeute: 45 %

7/3: trans-4-Nitroxycyclohexancarbonsäure-N-(N.N-dimethyl-acetamido)amid
aus trans-4-Nitroxycyclohexancarbonsäurechlorid und
Aminoessigsäure-N,N-dimethyl-amid
Schmelzpunkt: 142-144oC (Ethanol), Ausbeute: 20 %

7/4: trans-4-Nitroxycyclohexancarbonsäure-N-(2,2-dimethylacetamido)amid
aus trans-4-nitroxycyclohexancarbonsäurechlorid und 2-Amino-2-methyl-propionsäureamid
Schmelzpunkt: 180-181oC (Wasser), Ausbeute: 53 %

7/5: trans-4-Nitroxycyclohexancarbonsäure-N-(methyl)hydroxylamid
aus trans-4-Nitroxycyclohexancarbonsäurechlorid und
N-Methyl-hydroxylamin
Schmelzpunkt: 125-126oC (Ether), Ausbeute: 53 %

7/6: trans-4-Nitroxycyclohexancarbonsäure-N-2-(2-methyl-3-ethyl)amid
aus trans-4-Nitroxycyclohexancarbonsäurechlorid und
2-Amino-2-methyl-propanol
Schmelzpunkt: 150-151°C (Ether), Ausbeute 65 % d.Th.

7/7: trans-4-Nitroxycyclohexancarbonsäure-N-bis-(2-hydroxy-ethyl)amid
aus trans-4-Nitroxycyclohexancarbon-säurechlorid und
Bis-(2-hydroxyethyl)amin
Schmelzpunkt: 78-79°C (Essigester/Ether), Ausbeute 80 % d.Th.

Beispiel 8

trans-4-Nitroxycyclohexancarbonsäure-N-(essigsäure)amid

1.1 g (0.015 mol) Aminoessigsäure werden in 25 ml Wasser gelöst und 0.6 g (0.015 mol) Natriumhydroxyd zugegeben. Nach Abkühlen auf ca. 5°C wird eine Lösung von 3.1 g (0.015 mol) trans-4-Nitroxy- cyclohexancarbonsäurechlorid in 10 ml Dioxan zugetropft und durch gleichzeitige Zugabe von 2 N Natronlauge (ca. 7.5 ml) der pH-Wen bei ca. 12 gehalten. Man läßt 2 h bei Raumtemperatur nachrühren, stellt den pH-Wert der Reaktionsmischung auf 1 und extrahiert mit Essigester. Die organische Phase wird mit Natriumsulfat getrocknet und im Vacuum abdestilliert. Nach Anreiben mit Ether und Absaugen verbleiben 1.4 g der Titelverbindung vom Schmp. 143-144°C, d.s. 38 % d.Th..

In analoger Weise werden erhalten:

8/1: trans-4-Nitroxycyclohexancarbonsäure-N-(2-propion-säure)amid

aus trans-4-Nitroxycyclohexancarbonsäurechlorid und 2-Amino-propionsäure
Schmelzpunkt: 113-114°C (Ether), Ausbeute: 25 %

8/2: trans-4-Nitroxycyclohexancarbonsäure-N-(acetamido)amid
aus trans-4-Nitroxycyclohexancarbonsäurechlorid und Aminoessigsäureamid
Schmelzpunkt: 170°C (Essigester), Ausbeute: 35 %

Beispiel 9

2-Hydroxy-N-(trans-4-nitroxycyclohexyl)essigsäureamid

6.1 g (0.023 mol) 2-Acetoxy-N-(trans-4-Nitroxycyclohexyl)essig-äureamid (Beispiel 6/2) werden in 100 ml ca. 5 N methanolischem Ammoniak gelöst und über Nacht bei Raumtemperatur gerührt. Nach abdestillieren im Vacuum wird in Essigester gelöst und mit Wasser ausgeschüttelt. Die Essigesterphase wird mit Natriumsulfat getrocknet und im Vacuum abdestilliert. Der Rückstand wird mit Ether verrieben und abgesaugt. Es verbleiben 1.8 g der Titelverbindung vom Schmp. 81-83°C, d.s. 36 %d.Th..

Analog werden erhalten:

9/1: 2-Hydroxy-N-(trans-4-nitroxycyclohexyl)propionsäureamid
aus 2-Acetoxy-N-(trans-4-nitroxycyclohexyl)propionsäureamid (Beisp. 6/3)
Schmelzpunkt: 117-118°C (Ether), Ausbeute: 23 %

9/2: 2-Hydroxy-N-(trans-4-nitroxycyclohexyl)-2,2-dimethyl-propionsäuramid
aus 2-Acetoxy-N-(trans-4-nitroxycyclohexyl)-2,2-dimethyl-propionsäureamid (Beispl. 6/4) Schmelzpunkt: 100-101°C (Ether), Ausbeute: 28 %

9/3: 2-Hydroxy-N-(trans-4-nitroxycyclohexylmethyl)essig-säureamid
aus 2-Acetoxy-N-(trans-4-nitroxycyclohexyl-methyl)essig-säureamid (Beisp. 6/7)
Schmelzpunkt: 111-112°C (Ether), Ausbeute: 74 %

9/4: 2,2-Bis-(hydroxymethyl)-N-trans-4-nitroxycyclohexylpropionsäureamid
aus 2,2-Bis-(acetoxymethyl)-N-trans-4-nitroxycyclohexylpropionsäure
Schmelzpunkt: 110-112°C (Essigester), Ausbeute 20 % d.Th.

Beispiel 10

4-Hydroxy-N-(trans-4-nitroxycyclohexyl)buttersäureamid

4.8 g (0.03 mol) trans-4-Nitroxycyclohexylamin werden mit 5.7 ml 4-Butyrolacton gemischt und 3 d bei Raumtemperatur gerührt. Zur Entfernung des überschüssigem Lactons wird über eine Kieselgelsäure mit Essigester filtriert. Nach Sammeln der reinen Fraktionen wird im Vacuum abdestilliert mit Ether verrieben und abgesaugt. Es verbleiben 2.1 g der Titelverbindung vom Schmp. 58-60°C, d.s. 28 % d.Th..

Beispiel 11

N-(trans-4-Nitroxycyclohexyl)-(S)-pyroglutaminsäureamid

2.1 g (0.016 mol) (S)-Pyroglutaminsäure (2-Pyrrolidinon-5-carbonsäure) werden in 50 ml abs. Tetrahydrofuran und 2.5 ml (0.018 mol) Triethylamin gelöst. Unter Kühlen auf ca. 5°C werden 2 ml (0.016 mol) Pivaloylchlorid in 10 ml abs. Tetrahydrofuran zuge- tropft. Nach 15 min rühren bei 5°C, tropft man eine Lösung von 3.9 g trans-4-Nitroxycyclohexylamin in 40 ml abs. Tetrahydrofuran und 2.8 ml (0.02 mol) Triethylamin zu. Danach läßt man 2 d bei Raumtemperatur rühren, saugt ab und entfernt im Vacuum das Lösungsmittel. Der Rückstand wird in Essigester gelöst und mit Natriumhydrogencarbonat ausgeschüttelt. Nach Trocknen der Essigesterphase mit Natriumsulfat, Abdestillieren im Vacuum, Anreiben mit Ether und Absaugen, verbleiben 1.5 g der Titelverbindung vom Schmp. 166°C, d.S. 35% d.Th..

Beispiel 12

N-(trans-4-Nitroxycydohexyl)nicotinsäureamid-N-Oxid

1,5 g (0,005 mol) N-(trans-4-Nitroxycyclohexyl)nicotinsäureamid (Beisp. 6/5) werden in 4 ml Essigsäure gelöst, 4 ml 30proz. Wasserstoffperoxid zugegeben und 2d bei 40°C gerührt. Nach Einengen und Anreiben mit Essigester werden die Kristalle abgesaugt. Es verbleiben 0,9g der Titelverbindung vom Schmp. 170-171°C, d.S. 53% d.Th.

Analog erhält man:

12/1 N-(trans-4-Nitroxycyclohexyl)isonicotinsäureamid-N-Oxid
aus N-(trans-4-Nitroxycyclohexyl)isonicotinsäureamid (Beisp. 6/6).
Schmelzpunkt: 180°C (Essigester), Ausbeute: 82 %

Beispiel 13

trans-4-Hydroxycyclohexylmethylamin (VII)

a) cis-trans 4-Hydroxycyclohexancarbonsäure

184 g (1.07 mol) cis-trans 4-Hydroxycyclohexancarbonsäureethyl- ester (Lit.: JACS 70, (1948) 1898) werden in 1.8 1 Wasser mit 119.8 g (2.14 mol) Kaliumhydroxid 3 h am Rückfluß erhitzt. Nach Ansäuren mit conc. Salzsäure und extrahieren mit Methylenchlorid werden 146 g Säure vom Schmp. 111-115°C, d. s. 94 % d. Th. erhalten.

b) trans-4-0-Acetylcyclohexancarbonsäure

145 g (1.01 mol) cis-trans-4-Hydroxycyclohexancarbonsäure werden in 1 1 Essigsäure suspendiert, 123.5 g (1.21 mol) Acetylchlorid zugetropft und darauf 5 h am Rückfluß erhitzt. Nach Abdestillieren der Essigsäure werden ca. 200 ml Wasser zugegeben und zur Trockene abdestilliert. Der Rückstand wird in Diisopropylether aufgenommen und nach Kristallisation abgesaugt. Es verbleiben 112 g Rohprodukt. Nach Umkristallisieren am 740 ml Wasser werden 84 g, d. s. 44 % d. Th. reine trans Verbindung vom Schmp. 140-141°C erhalten.

c) trans-4-0-Acetylcyclohexancarbonsäuremethylester

84 g (0.45 mol) trans-4-0-Acetylcyclohexancarbonsäure werden in 1 1 Methanol gelöst, 8.6 g (0.045 mol) p-Toluolsulfonsäure zugegeben und etwa 20 h am Rückfluß erhitzt. Nach Abdestillieren des Methanols wird der Rückstand in Wasser gelöst und mit Natriumhydrogencarbonat neutralisiert. Man sättigt die wässrige Lösung mit Kochsalz und extrahiert mehrmals mit Essigester. Die Essigesterextrakte werden mit Natriumsulfat getrocknet, abfiltriert und abdestilliert. Es verbleiben 72.7 g Ester, d. s. 80 % d. Th. als farbloses Öl.

d) trans-4-Hydroxycyclohexancarbonsäureamid

36 g (0.18 mol) trans-4-0-Acetylcyclohexancarbonsäuremethylester werden in 500 ml Methanol und 500 ml flüssigem Ammoniak 24 h auf 100oC im 2 1 Schüttel-Autoklaven erhitzt. Nach Verdampfen des Ammoniaks und Abdestillieren des Methanols wird der Rückstand mit Ether verrieben und abgesaugt. Es verbleiben 21.6 g Amid vom Schmp. 208-210°C, d. s. 83 % d. Th.

e) trans-4-Hydroxycyclohexylmethylamin

Zu einer Suspension von 11.4 g (0.3 mol) Lithiumaluminiumhydrid in 500 ml wasserfreiem Tetrahydrofuran werden 21.6 g (0.15 mol) trans-4-Hydroxycyclohexancarbonsäureamid fest eingetragen und 24 h am Rückfluß erhitzt. Nach Zersetzen mit 45 ml gesättigter Kochsalzlösung wird abgesaugt und das Filtrat abdestilliert. Der Rückstand wird mit Ether verrieben und die Kristalle abgesaugt. Es verbleiben 11 g Amin, d. s. 57 % d. Th. vom Schmp. 137-139°C.

Beispiel 14

Pharmakologische Untersuchungen

a) Untersuchungsziel

Ziel der vorliegenden Untersuchungen war es, Nitrate zu finden, deren Wirkung länger anhält und die therapeutisch den Vorteil erwarten lassen, daß eine einmal tägliche Gabe für die therapeutische Anwendung genügt. Abgesehen davon, daß die einmalige tägliche Verabreichung anstelle einer mehrfachen eine Verbesserung für die Compliance des Patienten bedeutet, kann auf diese Weise auch das pharmakokinetische Wirkprofil einer Substanz wesentlich beeinflußt werden, d. h. man darf davon ausgehen, daß die Differenz zwischen Maximal- und Minimalspiegel wegen des geringeren Konzentrationsabfalls der Substanzspiegel deutlich günstiger ist.

b) Methode

Zum Nachweis der Denitrierung, die das Wirkprinzip aller Nitrate darstellt, wurde die Denitrierungsgeschwindigkeit im Verhältnis zu der des bekannten ISDN-Metaboliten IS-5M bewertet. Hierzu wurden Ratten in Narkose getötet und die Leber mit einer entsprecnenu konzentrierten äquimolaren ($5 \times 10^{-5}$ M/l) Lösung von IS-5-MN bzw. den zu prüfenden Substanzen jeweils 4 min reperfundiert und im Perfusat die freigesetzte Menge an NO2 bestimmt. Um vergleichende Bedingungen zu haben, wurde die Perfusion mit IS-5-MN (Standard-substanz) als Kontrolle dreimal so verabreicht als wäre die dritte Perfusion eine unbekannte Substanz (auf diese Weise kann eine unter den Versuchsbedingungen veränderte Leberleistung erkannt und entsprechend berücksichtigt werden). Die $V_{rel}$-Werte (relative Denitrierungsgeschwindigkeit), dritte Spalte der Tabelle, geben an, wie hoch die Denitrierungsgeschwindigkeit im Verhältnis zu IS-5-MN ist. Hohe Werte bedeuten schnelle Denitrierung, geringere Werte langsame Denitrierung.

c) Ergebnisse

Bezüglich der Denitrierungsgeschwindigkeit durch die perfundierte Rattenleber sind im Vergleich zu Isosorbit-5-mononitrat (IS-5-MN) mit $V_{rel}$=0.95 die untersuchten Verbindungen bessser, d. h. es finden sich kleinere Werte der Denitrierungsgeschwindigkeit als bei IS-5-MN.

Tabelle:

| Bsp. Nr. | Vrel |
|---|---|
| 1 | 0.45 |
| 5/3 | 0.49 |
| 7/7 | 0.49 |
| 10 | 0.28 |
| 12/1 | 0.36 |

**Patentansprüche**

**1.** Cyclohexanolnitrate der Formel I

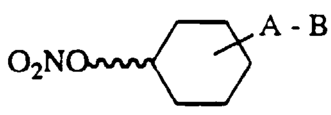

(I)

in der

A einen Valenzstrich oder eine $C_1$-$C_6$-Alkylenkette und
B die Gruppe -$NR^1$-CO-Z, $NR^1$-SO2-Z oder -CO-$NR^2$-Z bedeuten, wobei
$R^1$ Wasserstoff oder eine $C_1$-$C_6$-Alkylgruppe,
$R^2$ Wasserstoff, eine Hydroxy-, Hydroxy-$C_1$-$C_6$-alkyl-, $C_1$-$C_6$-Alkoxy-, $C_1$-$C_6$-Alkyl, $C_2$-$C_6$-Alkenyl- oder $C_2$-$C_6$-Alkinylgruppe und
Z Wasserstoff, eine $C_1$-$C_6$-Alkyl-, $C_2$-$C_6$-Alkenyl- oder $C_2$-$C_6$-Alkinylgruppe bedeutet, die ein- oder mehrfach durch eine Hydroxy-, $C_1$-$C_6$-Alkylcarbonyloxy-, $C_1$-$C_6$-Alkoxy-, Halogen-, Cyano-, Carboxy-, $C_1$-$C_6$-Alkoxycarbonyl, -CO-$NR^3R^4$, Mercapto-, $C_1$-$C_6$-Alkylmercapto- oder $C_1$-$C_6$-Alkylcar-

bonylmercaptogruppe substituiert sein können, oder Z eine $C_3$-$C_6$-Cycloalkylgruppe oder einen Pyridin-, N-Oxy-pyridin-, Tetrazolyl- oder Pyrrolidinonring darstellen, oder Z zusammen mit $R^2$ und dem Stickstoffatom, an das Z und $R^2$ gebunden sind, einen heterocyclischen Ring bildet, der zusätzlich ein Sauerstoff-, Schwefel- oder Stickstoffatom enthalten kann, wobei der heterocyclische Ring mit einem zusätzlichen Stickstoffatom gegebenenfalls am Stickstoffatom durch eine $C_1$-$C_6$-Alkylcarbonylgruppe acyliert sein kann, und für den Fall, daß B eine -$NR^1$-CO-Z- Gruppe ist, Z auch eine $C_1$-$C_6$-Alkoxygruppe bedeuten kann, oder wenn Z eine durch eine Mercapto-, $C_1$-$C_6$-Alkylmercapto- oder $C_1$-$C_6$-Alkylcarbonylmercaptogruppe substituierte $C_1$-$C_6$-Alkyl- oder $C_2$-$C_6$-Alkenylgruppe bedeutet, die $C_1$-$C_6$-Alkyl- oder $C_2$-$C_6$-Alkenylgruppe zusätzlich durch die Gruppe -$NR^4R^5$ oder eine $C_1$-$C_4$-Alkylcarbonylgruppe substituiert sein kann, und

$R^3$ und $R^4$ gleich oder verschieden sein können und jeweils unabhängig voneinander Wasserstoff, eine $C_1$-$C_6$-Alkylgruppe oder $R^3$ und $R^4$ zusammen mit dem Stickstoffatom, an das sie gebunden sind, einen heterocyclischen Ring bilden, der zusätzlich ein Sauerstoff-, Schwefel- oder Stickstoffatom enthalten kann, und $R^5$ Wasserstoff, Methyl oder Acetyl bedeutet,

sowie deren optisch aktive Formen und physiologisch verträgliche Salze.

**2.** Cyclohexanolnitrate gemäß Anspruch 1, dadurch gekennzeichnet, daß A einen Valenzstrich oder eine $C_1$-$C_3$-Alkylengruppe, insbesondere die -$CH_2$-Gruppe bedeutet.

**3.** Cyclohexanolnitrate gemäß Anspruch 1 oder 2, dadurch gekennzeichnet, daß $R^1$ ein Wasserstoffatom oder eine $C_1$-$C_3$-Alkylgruppe, insbesondere die Methyl- oder Ethylgruppe darstellt.

**4.** Cyclohexanolnitrate gemäß einem der Ansprüche 1-3, dadurch gekennzeichnet, daß $R^2$ ein Wasserstoffatom, eine Hydroxy-, Hydroxy-$C_1$-$C_3$-alkyl- oder $C_1$-$C_3$-Alkylgruppe, insbesondere die Methyl- oder Ethylgruppe, darstellt, oder $R^2$ und Z zusammen eine Morpholinogruppe bilden.

**5.** Cyclohexanolnitrate gemäß einem der Ansprüche 1-4, dadurch gekennzeichnet, daß $R^3$ und $R^4$ unabhängig voneinander ein Wasserstoffatom oder eine $C_1$-$C_3$-Alkylgruppe, insbesondere die Methylgruppe, darstellen.

**6.** Cyclohexanolnitrate gemäß einem der Ansprüche 1-5, dadurch gekennzeichnet, daß Z ein Wasserstoffatom, eine $C_1$-$C_6$-Alkyl, $C_2$-$C_6$-Alkenyl- oder eine $C_2$-$C_6$-Alkinylgruppe bedeutet, wobei die Alkyl- und Alkenylgruppen einfach substituiert sein können durch ein Halogenatom, eine Carboxy-, Carboxamido-, $C_1$-$C_3$-Alkoxy-, $C_1$-$C_3$-Alkylcarbonyloxy-, Hydroxy- oder Cyanogruppe, oder die Alkyl- und Alkenylgruppen zweifach substituiert sein können durch Hydroxy- oder $C_1$-$C_3$-Alkylcarbonyloxygruppen, oder Z eine 3- oder 4-Pyridylgruppe bzw. N-Oxy-pyridylgruppe oder den Pyrrolidinonring bedeutet, oder für den Fall, daß B eine -$NR^2$-CO-Z-Gruppe bedeutet, Z eine $C_1$-$C_3$-Alkoxygruppe bedeutet.

**7.** Cyclohexanolnitrate gemäß Anspruch 1 ausgewählt aus der Gruppe der folgenden Verbindungen:

trans-N-Acetyl-4-nitroxycyclohexylamin,
N-(trans-4-Nitroxycyclohexylmethyl)bernsteinsäurediamid,
trans-4-Nitroxycyclohexancarbonsäure-N-bis-(2-hydroxyethyl)amid,
4-Hydroxy-N-(trans-4-nitroxycyclohexyl)buttersäureamid,
N-(trans-4-Nitroxycyclohexyl)isonicotinsäureamid-N-Oxid.

**8.** Verfahren zur Herstellung von Cyclohexanolnitraten gemäß einem der Ansprüche 1-7, dadurch gekennzeichnet, daß man

1) eine Verbindung der allgemeinen Formel II,

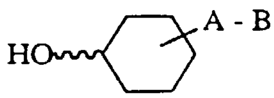

(II)

in der A und B die oben angegebenen Bedeutungen haben, einer Nitratester-Bildungsreaktion unterwirft,

2) oder eine Verbindung der allgemeinen Formel III,

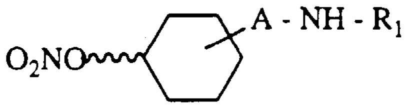

(III)

mit einer Verbindung der allgemeinen Formel IV,

W-CO-Z (IVa)

oder

$W-SO_2-Z$ (IVb)

wobei $R^1$ und Z die oben angegebenen Bedeutungen haben und W eine reaktionsfähige Gruppe darstellt, umsetzt, oder

3) eine Verbindung der allgemeinen Formel V,

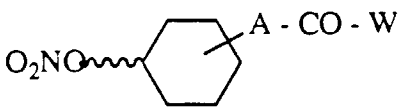

(V)

mit einer Verbindung der allgemeinen Formel VI,

$R^2$-NH-Z (VI)

wobei $R^2$, W, A und Z die angegebenen Bedeutungen haben, umsetzt,

und anschließend die so erhaltenen Verbindungen gegebenen falls nachträglich in andere Verbindungen der Formel I umwandelt oder gegebenenfalls in die physiologisch verträglichen Salze überführt.

**9.** Arzneimittel enthaltend Cyclohexanolnitrate gemäß einem der Ansprüche 1-7 neben pharmazeutisch üblichen Träger- oder Hilfsstoffen.

**10.** Verwendung von Verbindungen der Formel I gemäß einem der Ansprüche 1-7 zur Herstellung von Arzneimitteln zur Behandlung von Herz- und Kreislauferkrankungen.

**11.** Verbindungen der Formel II

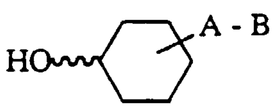

(II)

in denen

A einen Valenzstrich oder eine $C_1$-$C_6$-Alkylenkette und
B die Gruppe $-NR^1$-CO-Z, $-NR^1-SO_2$-Z oder $-CO-NR^2$-Z bedeuten, wobei
$R^1$ Wasserstoff oder eine $C_1$-$C_6$-Alkylgruppe,
$R^2$ Wasserstoff, eine Hydroxy-, Hydroxy-$C_1$-$C_6$-alkyl-, $C_1$-$C_6$-Alkoxy-, $C_1$-$C_6$-Alkyl-, $C_2$-$C_6$-Alkenyl- oder $C_2$-$C_6$-Alkinylgruppe und
Z eine $C_1$-$C_6$-Alkyl-, $C_2$-$C_6$-Alkenyl- oder $C_2$-$C_6$-Alkinylgruppe bedeutet, die ein- oder mehrfach durch eine Hydroxy-, $C_1$-$C_6$-Alkyl-carbonyloxy-, $C_1$-$C_6$-Alkoxy-, Halogen-, Cyano-, Carboxy-, $C_1$-$C_6$-Alkoxycarbonyl-, $-CO-NR^3R^4$-, Mercapto-, $C_1$-$C_6$-Alkylmercapto- oder $C_1$-$C_6$-Alkylcarbonylmercapto-Gruppe substituiert ist, oder einen Tetrazolyl- oder Pyrrolidinon-ring darstellt, oder
Z zusammen mit $R^2$ und dem Stickstoffatom, an das Z und $R^2$ gebunden sind, einen heterocyclischen Ring bildet, der zusätzlich ein Sauerstoff-, Schwefel- oder Stickstoffatom enthalten kann, wobei der heterocyclische Ring mit einem zusätzlichen Stickstoffatom am Stickstoffatom durch eine $C_1$-$C_6$-Alkylcarbonylgruppe acyliert sein kann, und für den Fall, daß Z eine durch eine Mercapto-, $C_1$-$C_6$-Alkylmercapto- oder $C_1$-$C_6$-Alkycarbonylmercaptogruppe substituierte $C_1$-$C_6$-Alkyl- oder Alkenylgruppe bedeutet, die $C_1$-$C_6$-Alkyl- oder Alkenylgruppe zusätzlich durch die Gruppe $-NR^4R^5$ oder eine $C_1$-$C_4$-Alkycarbonylgruppe substituiert sein kann, und

$R^3$ und $R^4$ gleich oder verschieden sein können und jeweils unabhängig voneinander Wasserstoff, eine $C_1$-$C_6$-Alkylgruppe oder $R^3$ und $R^4$ zusammen mit dem Stickstoffatom, an das sie gebunden sind, einen heterocyclischen Ring bilden, der zusätzlich ein Sauerstoff-, Schwefel- oder Stickstoffatom enthalten kann und

$R^5$ Wasserstoff, Methyl oder Acetyl bedeutet.

## Claims

1. Cyclohexanol nitrates of the formula I

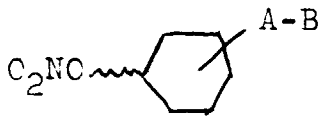

(I)

in which A signifies a valency bond or a $C_1$-$C_6$-alkylene chain and B the group -$NR^1$-CO-Z, $NR^1$-$SO_2$-Z or -CO-$NR^2$-Z, whereby $R^1$ signifies hydrogen or a $C_1$-$C_6$-alkyl group, $R^2$ hydrogen, a hydroxyl, hydroxy-$C_1$-$C_6$-alkyl, $C_1$-$C_6$-alkoxy, $C_1$-$C_6$-alkyl, $C_2$-$C_6$-alkenyl or $C_2$-$C_6$-alkynyl group and Z signifies hydrogen, a $C_1$-$C_6$-alkyl, $C_2$-$C_6$-alkenyl or $C_2$-$C_6$-alkynyl group which can be substituted one or more times by a hydroxyl, $C_1$-$C_6$-alkylcarbonyloxy, $C_1$-$C_6$-alkoxy, halogen, cyano, carboxyl, $C_1$-$C_6$-alkoxycarbonyl, -CO-$NR^3R^4$, mercapto, $C_1$-$C_6$-alkylmercapto or $C_1$-$C_6$-alkylcarbonylmercapto group or Z represents a $C_3$-$C_6$-cycloalkyl group or a pyridine, N-oxypyridine, tetrazolyl or pyrrolidinone ring or Z, together with $R^2$ and the nitrogen atom to which Z and $R^2$ are attached, forms a heterocyclic ring which can additionally contain oxygen, sulphur or nitrogen atom, whereby the heterocyclic ring with an additional nitrogen atom can possibly be acylated on the nitrogen atom by a $C_1$-$C_6$-alkylcarbonyl group and, for the case that B is an -$NR^1$-CO-Z group, Z can also signify a $C_1$-$C_6$-alkoxy group or when Z signifies a $C_1$-$C_6$-alkyl or $C_2$-$C_6$-alkenyl group substituted by a mercapto, $C_1$-$C_6$-alkylmercapto or $C_1$-$C_6$-alkylcarbonylmercapto group, the $C_1$-$C_6$-alkyl or $C_2$-$C_6$-alkenyl group can additionally be substituted by the group -$NR^4R^5$ or a $C_1$-$C_4$-alkylcarbonyl group and $R^3$ and $R^4$ can be the same or different and, in each case independently of one another, signify hydrogen, a $C_1$-$C_6$-alkyl group or $R^3$ and $R^4$, together with the nitrogen atom to which they are attached, form a heterocyclic ring which can additionally contain an oxygen, sulphur or nitrogen atom and $R^5$ signifies hydrogen, methyl or acetyl, as well as their optically-active forms and physiologically compatible salts.

2. Cyclohexanol nitrates according to claim 1, characterised in that A signifies a valency bond or a $C_1$-$C_3$-alkylene group, especially the -$CH_2$- group.

3. Cyclohexanol nitrates according to claim 1 or 2, characterised in that $R^1$ represents a hydrogen atom or a $C_1$-$C_3$-alkyl group, especially the methyl or ethyl group.

4. Cyclohexanol nitrates according to one of claims 1 - 3, characterised in that $R^2$ represents a hydrogen atom, a hydroxyl, hydroxy-$C_1$-$C_3$-alkyl or $C_1$-$C_3$-alkyl group, especially the methyl or ethyl group, or $R^2$ and Z together form a morpholino group.

5. Cyclohexanol nitrates according to one of claims 1 - 4, characterised in that $R^3$ and $R^4$, independently of one another, represent a hydrogen atom or a $C_1$-$C_3$-alkyl group, especially the methyl group.

6. Cyclohexanol nitrates according to one of claims 1 - 5, characterised in that Z signifies a hydrogen atom, a $C_1$-$C_6$-alkyl, $C_2$-$C_6$-alkenyl or a $C_2$-$C_6$-alkynyl group, whereby the alkyl and alkenyl groups can be substituted once by a halogen atom, a carboxyl, carboxamido, $C_1$-$C_3$-alkoxy, $C_1$-$C_3$-alkylcarbonyloxy, hydroxyl or cyano group, or the alkyl and alkenyl groups can be substituted twice by hydroxyl or $C_3$-$C_3$-alkylcarbonyloxy groups or Z signifies a 3- or 4-pyridyl group or N-oxypyridyl group or the pyrrolidinone ring or, for the case that B signifies an -$NR^2$-CO-Z group, Z signifies a $C_1$-$C_3$-alkoxy group.

7. Cyclohexanol nitrates according to claim 1 selected from the group of the following compounds:

trans-N-acetyl-4-nitroxycyclohexylamine,
N-(trans-4-nitroxycyclohexylmethyl)-succinic acid diamide,
trans-4-nitroxycyclohexanecarboxylic acid N-bis-(2-hydroxyethyl)-amide,
4-hydroxy-N-(trans-4-nitroxycyclohexyl)-butyric acid amide,

N-(trans-4-nitroxycyclohexyl)-isonicotinic acid amide N-oxide.

**8.** Process for the preparation of cyclohexanol nitrates according to one of claims 1 - 7, characterised in that one

1) subjects a compound of the general formula II

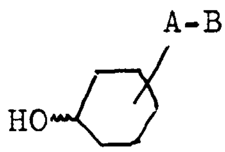

(II)

in which A and B have the above-given meanings, to a nitrate ester formation reaction,
2) or reacts a compound of the general formula III

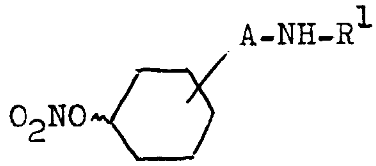

(III)

with a compound of the general formula IV,

W-CO-Z (IVa)

or

$W\text{-}SO_2\text{-}Z$ (IVb)

whereby $R^1$ and Z have the above-given meanings and W represents a reactive group, or
3) reacts a compound of the general formula V,

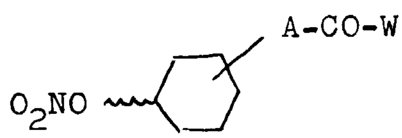

(V)

with a compound of the general formula VI,

$R^2NH\text{-}Z$ (VI)

whereby $R^2$, W, A and Z have the given meanings, and subsequently possibly thereafter converts the so obtained compounds into other compounds of the formula I or possibly converts into the physiologically compatible salts.

**9.** Medicaments containing cyclohexanol nitrates according to one of claims 1 - 7, besides pharmaceutically usual carriers or adjuvant materials.

**10.** Use of compounds of the formula I according to one of claims 1 - 7 for the production of medicaments for the treatment of heart and circulatory diseases.

**11.** Compounds of the formula II

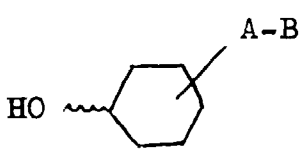

(II)

in which A signifies a valency bond or a $C_1$-$C_6$-alkylene chain and B the group $-NR^1\text{-}CO\text{-}Z$, $-NR^1\text{-}SO_2\text{-}Z$ or $-CO\text{-}NR^2\text{-}Z$, whereby $R^1$ signifies hydrogen or a $C_1$-$C_6$-alkyl group, $R^2$ hydrogen, a hydroxyl, hydroxy-$C_1$-$C_6$-alkyl, $C_1$-$C_6$-alkoxy, $C_1$-$C_6$-alkyl, $C_2$-$C_6$-alkenyl or $C_2$-$C_6$-alkynyl group or Z signifies a $C_1$-$C_6$-alkyl, $C_2$-$C_6$-alkenyl or $C_2$-$C_6$-alkynyl group which is substituted one or more times by a hydroxyl, $C_1$-$C_6$-alkylcarbonyloxy, $C_1$-$C_6$-alkoxy, halogen, cyano, carboxyl, $C_1$-$C_6$-alkoxycarbonyl, $-CO\text{-}NR^3R^4$, mercapto, $C_1$-$C_6$-alkylmercapto or $C_1$-$C_6$-alkylcarbonylmercapto group, or represents a tetrazolyl or pyrrolidinone ring, or Z, together with $R^2$ and the nitrogen atom to which

Z and $R^2$ are attached, forms a heterocyclic ring which can additionally contain an oxygen, sulphur or nitrogen atom, whereby the heterocyclic ring with an additional nitrogen atom can be acylated on the nitrogen atom by a $C_1$-$C_6$ alkylcarbonyl group, and, for the case that Z signifies a $C_1$-$C_6$-alkyl pr alkenyl group substituted by a mercapto, $C_1$-$C_6$-alkylmercapto or $C_1$-$C_6$-alkylcarbonylmercapto group, the $C_1$-$C_6$-alkyl or alkenyl group can be additionally substituted by the group $-NR^4R^5$ or a $C_1$-$C_4$-alkylcarbonyl group and $R^3$ and $R^4$ can be the same or different and, in each case independently of one another, signify hydrogen, a $C_1$-$C_6$-alkyl group or $R^3$ and $R^4$, together with the nitrogen atom to which they are attached, form a heterocyclic ring which can additionally contain an oxygen, sulphur or nitrogen atom and $R^5$ signifies hydrogen, methyl or acetyl.

**Revendications**

**1.** Nitrate de cyclohexanol de formule I

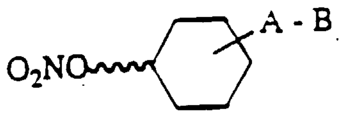

(I)

dans laquelle

A représente une valence libre ou une chaîne alkylène en $C_1$-$C_6$, et

B représente le groupe $-NR^1-CO-Z$, $-NR^1-SO_2-Z$ ou $-CO-NR^2-Z$, où

$R^1$ représente un atome d'hydrogène ou un groupe alkyle en $C_1$-$C_6$,

$R^2$ représente un atome d'hydrogène, un groupe hydroxy, hydroxy(alkyle en $C_1$-$C_6$), alcoxy en $C_1$-$C_6$, alkyle en $C_1$-$C_6$, alcényle en $C_2$-$C_6$ ou alcinyle en $C_2$-$C_6$, et

Z représente un atome d'hydrogène, un groupe alkyle en $C_1$-$C_6$, alcényle en $C_2$-$C_6$ ou alcinyle en $C_2$-$C_6$, qui peut porter un ou plusieurs substituants qui peuvent être un groupe hydroxy, un groupe (alkyle en $C_1$-$C_6$)-carbonyloxy, alcoxy en $C_1$-$C_6$, halogéno, cyano, carboxy, (alcoxy en $C_1$-$C_6$)-carbonyle, $-CO-NR^3NR^4$, mercapto, (alkyle en $C_1$-$C_6$)-mercapto ou (alkyle en $C_1$-$C_6$)-carbonylmercapto, ou Z représente un groupe cycloalkyle en $C_3$-$C_6$ ou un cycle pyridine, N-oxypyridine, tétrazolyle ou pyrrolidinone, ou Z forme, conjointement avec $R^2$ et l'atome d'azote auquel Z et $R^2$ sont liés, un hétérocycle, qui peut contenir un atome d'oxygène, de soufre ou d'azote supplémentaire, l'hétérocycle comportant un atome d'azote supplémentaire pouvant être acylé éventuellement sur l'atome d'azote au moyen d'un groupe (alkyle en $C_1$-$C_6$)-carbonyle, et dans le cas où B est un groupe $-NR^1-CO-Z-$, Z peut également représenter un groupe alcoxy en $C_1$-$C_6$, ou lorsque Z représente un groupe alkyle en $C_1$-$C_6$ ou alcényle en $C_2$-$C_6$ portant un substituant mercapto, (alkyle en $C_1$-$C_6$)-mercapto ou (alkyle en $C_1$-$C_6$)-carbonylmercapto, le groupe alkyle en $C_1$-$C_6$ ou alcényle en $C_2$-$C_6$ peut porter un substituant supplémentaire qui est le groupe $-NR^4R^5$ ou un groupe (alkyle en $C_1$-$C_4$)-carbonyle, et

$R^3$ et $R^4$ peuvent être identiques ou différents, et représentent chacun, indépendamment l'un de l'autre, un atome d'hydrogène, un groupe alkyle en $C_1$-$C_6$ ou $R^3$ et $R^4$ forment, conjointement avec l'atome d'azote auquel ils sont liés, un hétérocycle qui peut comporter un atome d'oxygène, de soufre ou d'azote supplémentaire, et

$R^5$ représente un atome d'hydrogène, un groupe méthyle ou acétyle,

ainsi que leurs formes optiquement actives et leurs sels physiologiquement acceptables.

**2.** Nitrate de cyclohexanol selon la revendication 1, caractérisé par le fait que A représente une valence libre ou un groupe alkylène en $C_1$-$C_3$, en particulier le groupe $-CH_2-$.

**3.** Nitrate de cyclohexanol selon la revendication 1 ou 2, caractérisé par le fait que $R^1$ représente un atome d'hydrogène ou un groupe alkyle en $C_1$-$C_3$, en particulier le groupe méthyle ou éthyle.

**4.** Nitrate de cyclohexanol selon l'une quelconque des revendications 1 à 3, caractérisé par le fait que $R^2$ représente un atome d'hydrogène, un groupe hydroxy ou hydroxy(alkyle en $C_1$-$C_3$) ou alkyle en $C_1$-$C_3$, en particulier le groupe méthyle ou éthyle, ou $R^2$ et Z forment ensemble un groupe morpholino.

**5.** Nitrate de cyclohexanol selon l'une quelconque des revendications 1 à 4, caractérisé par le fait que $R^3$ et $R^4$ représentent, indépendamment l'un de l'autre, un atome d'hydrogène ou un groupe alkyle en $C_1$-$C_3$, en particulier le groupe méthyle.

**6.** Nitrate de cyclohexanol selon l'une quelconque des revendications 1 à 5, caractérisé par le fait que Z représente un atome d'hydrogène, un groupe alkyle en $C_1$-$C_6$, alcényle en $C_2$-$C_6$ ou alcinyle en $C_2$-$C_6$, les groupes alkyle et alcényle pouvant porter un substituant qui peut être un atome d'halogène, un groupe carboxy, carboxamido, alcoxy en $C_1$-$C_3$, (alkyle en $C_1$-$C_3$)-carbonyloxy, hydroxy ou cyano, ou les groupes alkyle et alcényle peuvent porter deux substituants qui peuvent être des groupes hydroxy ou (alkyle en $C_1$-$C_3$)-carbonyloxy, ou Z représente un groupe 3- ou 4-pyridyle ou N-oxypyridyle, ou le cycle pyrrolidinone, ou dans le cas où B représente un groupe -N$R^2$-CO-Z-, Z représente un groupe alcoxy en $C_1$-$C_3$.

**7.** Nitrate de cyclohexanol selon la revendication 1, choisi dans le groupe constitué par les composés suivants :

trans-N-acétyl-4-nitroxycyclohexylamine,
diamide N-(trans-4-nitroxycyclohexylméthyl)succinique,
N-bis-(2-hydroxyéthyl)amide de l'acide trans-4-nitroxycyclohexanecarboxylique,
amide 4-hydroxy-N-(trans-4-nitroxycyclohexyl)butyrique,
N-oxyde de l'amide N-(trans-4-nitrocyclohexyl)isonicotinique.

**8.** Procédé de préparation de nitrate de cyclohexanol tel que défini dans l'une quelconque des revendications 1-7, caractérisé par le fait que

1) on soumet un composé de formule générale II

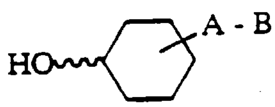

(II)

dans laquelle A et B ont les significations mentionnées plus haut, à une réaction de formation de nitrate-ester,

2) ou on fait réagir un composé de formule générale II

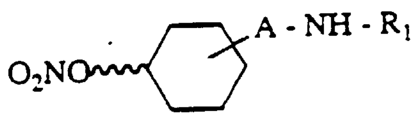

(III)

avec un composé de formule générale IV

W-CO-Z (IVa)

ou

W-$SO_2$-Z (IVb)

où $R^1$ et Z ont les significations mentionnées ci-dessus et W représente un groupe réactif, ou

3) on fait réagir un composé de formule générale V

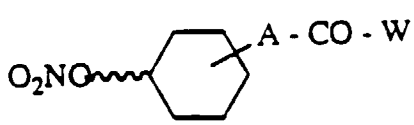

(V)

avec un composé de formule générale VI

$R^2$-NH-Z (VI)

où $R^2$, W, A et Z ont les significations mentionnées ci-dessus,

et ensuite, on transforme éventuellement les composés ainsi obtenus en d'autres composés de formule ou éventuellement en leurs sels physiologiquement acceptables.

**9.** Médicament contenant un nitrate de cyclohexanol selon l'une quelconque des revendications 1 à 7, en plus de véhicules ou d'adjuvants pharmaceutiques usuels.

**10.** Utilisation de composés de formule I selon l'une quelconque des revendications 1 à 7, pour la préparation de médicaments destinés au traitement de maladies cardiovasculaires.

**11.** Composés de formule II

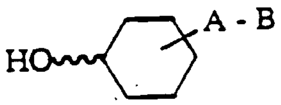

(II)

dans laquelle

A représente une valence libre ou une chaîne alkylène en $C_1$-$C_6$, et

B représente le groupe $-NR^1-CO-Z$, $-NR^1-SO_2-Z$ ou $-CO-NR^2-Z$, où

$R^1$ représente un atome d'hydrogène ou un groupe alkyle en $C_1$-$C_6$,

$R^2$ représente un atome d'hydrogène, un groupe hydroxy, hydroxy(alkyle en $C_1$-$C_6$), alcoxy en $C_1$-$C_6$, alkyle en $C_1$-$C_6$, alcényle en $C_2$-$C_6$ ou alcinyle en $C_2$-$C_6$, et

Z représente un groupe alkyle en $C_1$-$C_6$, alcényle en $C_2$-$C_6$ ou alcinyle en $C_2$-$C_6$, qui peut porter un ou plusieurs substituants qui peuvent être un groupe hydroxy, un groupe (alkyle en $C_1$-$C_6$)-carbonyloxy, alcoxy en $C_1$-$C_6$, halogéno, cyano, carboxy, (alcoxy en $C_1$-$C_6$)-carbonyle, $-CO-NR^3NR^4$, mercapto, (alkyle en $C_1$-$C_6$)-mercapto ou (alkyle en $C_1$-$C_6$)-carbonylmercapto, ou un cycle tétrazolyle ou pyrrolidinone, ou

Z forme, conjointement avec $R^2$ et l'atome d'azote auquel Z et $R^2$ sont liés, un hétérocycle, qui peut contenir en outre un atome d'oxygène, de soufre ou d'azote, l'hétérocycle comportant un atome d'azote supplémentaire pouvant être acylé éventuellement sur l'atome d'azote au moyen d'un groupe (alkyle en $C_1$-$C_6$)-carbonyle, et dans le cas où Z représente un groupe alkyle en $C_1$-$C_6$ ou alcényle, portant un substituant mercapto, (alkyle en $C_1$-$C_6$)-mercapto ou (alkyle en $C_1$-$C_6$)-carbonylmercapto, le groupe alkyle en $C_1$-$C_6$ ou alcényle peut porter un substituant supplémentaire qui peut être le groupe $-NR^4R^5$ ou un groupe (alkyle en $C_1$-$C_4$)-carbonyle, et

$R^3$ et $R^4$ peuvent être identiques ou différents, et représentent chacun, indépendamment l'un de l'autre, un atome d'hydrogène, un groupe alkyle en $C_1$-$C_6$ ou $R^3$ et $R^4$ forment, conjointement avec l'atome d'azote auquel ils sont liés, un hétérocycle qui peut comporter un atome d'oxygène, de soufre ou d'azote supplémentaire, et

$R^5$ représente un atome d'hydrogène, un groupe méthyle ou acétyle.